# EUROPÄISCHE PATENTSCHRIFT

(11) **EP 1 381 380 B1**
(45) Veröffentlichungstag und Bekanntmachung des Hinweises auf die Patenterteilung: **02.01.2008**
(21) Anmeldenummer: 02766644.5
(22) Anmeldetag: 26.04.2002
(51) Int. Cl.: A61K 36/898, A61P 9/00

(54) **PHARMAZEUTISCHE ZUSAMMENSETZUNG ENTHALTEND BULBOPHYLLUM**
PHARMACEUTICAL COMPOSITION CONTAINING BULBOPHYLLUM
COMPOSITION PHARMACEUTIQUE CONTENANT DU BULBOPHYLLUM

(30) Priorität: 26.04.2001 DE 10120630
(43) Veröffentlichungstag der Anmeldung: 21.01.2004
(73) Patentinhaber: Barthel, Michael, FL-9485 Nendeln (LI)
(72) Erfinder: Barthel, Michael, FL-9485 Nendeln (LI)
(74) Vertreter: Kuhnen & Wacker
(86) Internationale Anmeldenummer: PCT/EP2002/004655
(87) Internationale Veröffentlichungsnummer: WO 2002/087601

(56) Entgegenhaltungen:
- EP-A- 0 627 213
- DATABASE BIOSIS [Online] BIOSCIENCES INFORMATION SERVICE, PHILADELPHIA, PA, US; Dezember 1998 (1998-12) HSIEH WEN-TSONG ET AL: "The effects of bioactive components from Nervilia purpurea on isolated rabbit aorta." Database accession no. PREV199900150556 XP002209825 & CHINESE PHARMACEUTICAL JOURNAL (TAIPEI), Bd. 50, Nr. 6, Dezember 1998 (1998-12), Seiten 351-360, ISSN: 1016-1015
- DATABASE WPI Section Ch, Week 199519 Derwent Publications Ltd., London, GB; Class B07, AN 1995-145414 XP002209826 & JP 07 070942 A (KAWANO MERIKURON KK), 14. März 1995 (1995-03-14)

## Beschreibung

Die vorliegende Erfindung betrifft die Verwendung pharmazeutischer Zusammensetzungen mit einem Gehalt an Bulbophyllum, insbesondere *Bulbophyllum neilgherense,* zur Behandlung von Krankheiten des Herz-Kreislaufsystems gemäß Ansprüch 1.

Es besteht ein großer Bedarf für die Bereicherung der Therapie von Krankheiten des Herz-Kreislaufsystems Arzneistoffe, bzw. Arzneimittel bereitzustellen, die nicht nur eine gute Wirksamkeit besitzen, sondern auch eine große Indikationsbreite sowie geringe oder keine Nebenwirkungen.

Diese Aufgabe wurde in überraschender Weise durch die Bereitstellung der unten beschriebenen Bulbophyllum-haltigen pharmazeutischen Zusammensetzungen erfolgreich gelöst.

US-4,691,472 betrifft die Kultur von Epiphyten, insbesondere Orchideen. Dabei ist Bulbophyllum als Beispiel für eine Orchideengattung genannt.

Bulbophyllum-Extrakte sind bisher nur aus der EP-0 627 213 bekannt. Diese beschreibt eine kosmetische Formulierung zur Förderung des Haarwuchses, die einen aus Orchideenpflanzen, u.a. aus Bulbophyllum, gewonnenen Extrakt enthält.

Aus der EP 0 627 213 ist ein Haarwuchsmittel bekannt, das als aktiven Inhaltstoff einen aus einer Orchidee, wie z. B. Bulbophyllum, hergestellten Pflanzenextrakt enthält.

Aus dem Artikel *"*Folk Medicinal Plants of the Nagas in India" aus Asian Folklore Studies, Volume 58, 1999: 205 bis 230 ist die Verwendung von *Bulbophyllum Rothschildianum* als Paste zur Behandlung von Knochenfrakturen und Verstauchungen sowie als Hämostatikum bekannt.

In dem Artikel "Some Useful Rare and Endemic Plants of the Southem Western Ghats" aus J. Econ. Taxon Bot, Volume 26, Nr. 1 (2002) wird über einige nützliche, seltene und endemische Pflanzen wie z. B. Bulbophyllum neilgherrense und deren orale Verwendung zur Behandlung von Leukoderma berichtet.

Im Lichte dieses Standes der Technik ist der Befund, dass die Pflanzenspezies Bulbophyllum die unten beschriebene pharmakologische Aktivität aufweist, die sie zur Therapie von Krankheiten des Herz-Kreislaufsystems geeignet macht, unerwartet.

Die pharmakologischen Testergebnisse, aus denen diese Schlussfolgerungen gezogen werden mussten, sowie die konkreten Herz-Kreislaufindikationen werden in der Beschreibung im einzelnen dargelegt und erläutert.

*Bulbophyllum neilgherense* kommt aus der Familie der Orchidaceae. Es wächst als Epiphyt auf Laubbäumen, insbesondere auf den höheren in den Regenwäldern. Das Rhizom ist dick und wächst kriechend. Der Pseudobulbus ist 3-5 · 2-2,5 cm groß, konisch-eiförmig und endet in einem Solitärblatt. Das Blatt ist 4-11 · 1,8-2,2 cm groß, elliptisch-länglich und verengt sich an der Basis. Die Blüten sind braun und können ins violette gehen und sind in einer 7-12 cm langen Traubenrispe, die von der Basis der Pseudobulbus ausgeht. Die Blütenstiele sind 3-4 mm lang. Das Hochblatt in den Blütenständen ist 5-6 mm lang, verkehrt lanzettlich. Die dorsalen Kelchblätter sind 4-5 mm lang, sind oval und konkav. Die lateralen Kelchblätter sind 7-8 cm lang, sind sichelförmig, sind mit ihrem inneren Rand zusammengewachsen und bilden eine konkave kahnförmige Struktur. Die Blütenblätter sind 3-3,5 mm lang, dreieckig-oval. Die Blütenlippen sind 5-6 mm lang, rötlich, dreigelappt; der mittlere Lappen ist länger, oval-lanzettlich, die lateralen sind linear. Es gibt 4 Staubbeutel, die wächsern aussehen. Die Pflanze ist in Süd-Westindien häufig und allgemein verbreitet.

Für die erfindungsgemäßen Drogen wird bevorzugt der Hauptteil der Pflanze, nämlich der ganze Bulbus ohne die anhängenden Wurzeln und ohne das nach oben herausragende Blatt verwendet.

Unter Drogen versteht man getrocknete bzw. aufbereitete Pflanzenteile (pflanzliche Drogen) die zur Herstellung von Arzneizubereitungen verwendet werden. Man rechnet hierzu außerdem bestimmte aus diesen gewonnene Rohprodukte (z.B. fette und ätherische Öle, Harze, Balsame und Gummen).

Drogenzubereitungen mit angereichertem Wirkstoffgehalt schließen z.B. Extrakte, Tinkturen, Perkolate, Mazerate, Infuse, Preßsäfte, Dektote (Tee), Destillate ein. Unter Drogeninhaltsstoffen versteht man die Summe der für eine Droge charakteristischen chemischen Bestandteile, die therapeutisch wirksamen wie die therapeutisch selbst nicht wirksamen Begleit- oder Ballaststoffe wie z.B. Cellulose, Stärke, Wachse. Letztere bilden im allgemeinen den überwiegenden Teil an Extrastoffen.

Um für die Herstellung von Drogen geeignet zu sein, kann die Pflanze entweder aus ihrem natürlichen Vorkommen gewonnen werden oder artifiziell auf andere Bäume aufgepflanzt werden oder in Gewächshäusern gezogen werden.

Zur Aufbereitung der Pflanze werden die üblichen Verfahren, wie z.B. Reinigungs-, Zerkleinerungs- und Trocknungs- und Stabilisierungsverfahren verwendet. Unter den Trocknungsverfahren ist insbesondere die Gefriertrocknung zu erwähnen.

Da sich die Pflanze aber auch die Droge auch bei längerem Transport und auch in tropischer Wärme frisch hält, ist es möglich, sie später zu einer Gefriertrocknung zu bringen und dann weiterzuverarbeiten.

Die Zubereitung der pharmazeutischen Zusammensetzung bzw. der Droge erfolgt auf unterschiedliche Weise mittels bekannter Verfahren, bevorzugt als Verreibung mit Milchzucker oder als Alkohol-Extrakt. Im Falle der Zubereitung als Alkohol-Extrakt liegt der Alkoholgehalt zwischen 80 und 100 Vol.-%, bevorzugt 94 bis 98 Vol.-%, insbesondere bevorzugt 96 Vol.-%.

Der Drogengehalt pro Einzelgabe in dem Extrakt oder der Verreibung mit Milchzucker liegt bevorzugt im Bereich von 1 Nanogramm bis 1 Milligramm, insbesondere bevorzugt 10 Nanogramm bis 1 Mikrogramm.

Die Dosierung erfolgt bevorzugt 1-3 mal täglich über einen Zeitraum von 1 bis 12 Monaten, vorzugsweise 2 bis 5 Monaten, insbesondere bevorzugt 3 bis 4 Monaten. Es kann aber auch die Verabreichung von Einzelgaben in einem Abstand von 1 Tag bis 12 Monaten, bevorzugt 3 bis 4 Monaten ausreichend sein, um die erfindungsgemäße Wirkung zu erzielen.

Die zum Erreichen einer optimalen therapeutischen Wirkung geeignete Dosierung kann vom Fachmann leicht festgestellt und entsprechend angepasst werden.

### Therapeutische Darreichungsformen

Die Herstellung von pharmazeutischen Zusammensetzungen mit einem Gehalt an Bulbophyllum bzw. deren Einsatz bei der erfindungsgemäßen Verwendung erfolgt in üblicher Weise mittels geläufigen pharmazeutisch-technologischen Verfahren.

Dazu die Droge, d.h. bevorzugt der Extrakt oder die Verreibung mit Milchzucker, zusammen mit geeigneten, pharmazeutisch annehmbaren Hilfs- und Trägerstoffen zu den für die verschiedenen Indikationen und Applikationsorten geeigneten Arzneiformen verarbeitet.

Eine wichtige systemische Applikationsform ist die perorale Verabreichung als Tabletten, Hart- oder Weichgelatinekapseln, Dragees, Pulver, Pellets, Mikrokapseln, Oblongkomprimate, Granulate, Kautabletten, Lutschpastillen, Kaugummi, Sachets oder Globuli.

Als Hilfsstoffe für die Herstellung von pharmazeutischen Zusammensetzungen zur peroralen Verabreichung werden beispielsweise Gegenklebe- und Schmier- und Trennmittel, Dispergiermittel, wie z.B. flammendisperses Siliciumdioxid, Sprengmittel, wie z.B. verschiedene Stärkearten, PVP, Celluloseester als Granuliermittel, wie z.B. wachsartige und oder polymere Stoffe auf Euthragit®, Cellulose oder Cremophor®-Basis eingesetzt.

Antioxidantia, Süßungsmittel, wie z.B. Saccharose, Xylit oder Mannit, Geschmackskorrigentien, Aromastoffe, Konservierungsmittel, Farbstoffe, Puffersubstanzen, Direktappliziermittel, wie z.B. mikrokristalline Cellulose, Stärke und Stärkehydrolisate (z.B. Celutab®), Milchzucker, Polyethylenglykole, Polyvinylpyrrolidon und Dicalciumphosphat, Gleitmittel, Füllstoffe, wie z.B. Lactose oder Stärke, Bindemittel in Form von Lactose, Stärkearten, wie z.B. Weizen oder Mais bzw. Reisstärke, Cellulosederivate, wie z.B. Methylcellulose, Hydroxypropylcellulose oder Kieselerde, Talkum, Stearate, wie z.B. Magnesiumstearat, Aluminiumstearat, Calciumstearat, Talk, siliconisierter Talk, Stearinsäure, Cetylalkohol, hydrierte Fette, können ebenfalls verwendet werden.

Zur herkömmlichen Applikation auf der Haut lassen sich die üblichen Emulsionen, Gele, Salben, Cremes oder mischphasige bzw. amphiphile Emulsionssysteme (Öl/Wasser-Wasser/Öl-Mischphase) sowie Liposomen und Transfersomen nennen. Diese können bevorzugt auch epidural angewendet werden.

Als Hilfs- bzw. Trägerstoffe eignen sich beispielsweise Natriumalginat als Gelbildner zur Herstellung einer geeigneten Grundlage oder Cellulosederivate, wie z.B. Guar- oder Xanthangummi, anorganische Gelbildner, wie z.B. Aluminiumhydroxide oder Bentonite (sogenannte thixotrope Gelbildner), Polyacrylsäurederivate, wie z.B. Carbopol®, Polyvinylpyrrolidon, mikrokristalline Cellulose oder Carboxymethylcellulose. Weiterhin kommen amphyphile nieder- und höhermolekulare Verbindungen, wie Phospholipide in Betracht. Die Gele können entweder als Hydrogele auf Wasserbasis oder als hydrophobe Organogele, beispielsweise auf Basis von Gemischen nieder- und hochmolekularer Paraffinkohlenwasserstoffe und Vaseline vorliegen.

Als Emulgatoren lassen sich anionische, kationische oder neutrale Tenside, beispielsweise Alkaliseifen, Metallseifen, Aminseifen, sulfurierte und sulfonierte Verbindungen, Invertseifen, hohe Fettalkohole, Partialfettsäurester des Sorbitans und Polyoxyethylensorbitans, Wollwachs, Lanolin oder andere synthetische Produkte zur Herstellung der Öl/Wasser- und/oder Wasser/Öl-Emulsionen einsetzen.

Die hydrophilen Organogele können beispielsweise auf Basis hochmolekularer Polyethylenglykole zubereitet werden. Diese gelartigen Formen sind abwaschbar. Als Lipide in Form fett- und/oder öl- und/oder wachsartiger Komponenten zur Herstellung der Salben, Cremes oder Emulsionen werden Vaseline, natürliche oder synthetische Wachse, Fettsäuren, Fettalkohole, Fettsäureester, zum Beispiel als Mono-, Di- oder Triglyceride, Paraffinöl oder vegetabilische Öle, gehärtetes Rizinusöl oder Kokosöl, Schweinefett, synthetische Fette, z.B. auf Capryl-, Caprin-, Laurin- und Stearinsäurebasis, oder Triglyceridmischungen wie Miglycol® eingesetzt.

Zur Einstellung des pH-Wertes können osmotisch wirksame Säuren und Laugen, z.B. Salzsäure, Zitronensäure, Natronlauge, Kalilauge, Natriumhydrogencarbonat, ferner Puffersysteme, wie z.B. Citrat, Phosphat, Tris-Puffer oder Triethanolamin, verwendet werden.

Zur Erhöhung der Stabilität können noch Konservierungsmittel, z.B. Methyl oder Propylbenzoat- (Parabene) oder Sorbinsäure hinzugesetzt werden.

Als weitere topisch applizierbare Formen lassen sich Pasten, Puder oder Lösungen erwähnen. Die Pasten enthalten als konsistenzgebende Grundlage häufig lyophile und hydrophile Hilfsstoffe mit sehr hohem Feststoffanteil. Die Puder oder topisch applizierbaren Pulver können zur Erhöung der Dispersität sowie des Fließ- und Gleitvermögens sowie zur Verhinderung von Agglomeraten, z.B. Stärkearten, wie Weizen- oder Reisstärke, flammendisperses Siliciumdioxid oder Kieselerden, die auch als Verdünnungsmittel dienen, enthalten.

Als transdermale Systeme lassen sich insbesondere Pflaster herstellen, die auf Basis verschiedener Schichten und/oder Mischungen geeigneter Hilfs- und Trägerstoffe den Wirkstoff in gesteuerter Weise über längere oder kürzere Zeiträume abzugeben vermögen. Bei der Herstellung derartiger transdermaler Systeme kommen zum Zwecke einer verbesserten und/oder beschleunigten Penetration die Membrandurchdringung erhöhende Substanzen bzw. Permeationspromotoren, wie z.B. Ölsäure, Arzone®, Adipinsäurederivate, Ethanol, Harnstoff, Propylglycol neben geeigneten Hilfs- und Trägerstoffen, wie Lösungsmitteln, polymeren Bestandteilen, z.B. auf Basis von Eutragit®, in Betracht.

Die Pflaster werden vorzugsweise auf die Präcordialgegend aufgebracht.

Des weiteren können auch Injektabila verabreicht werden. Diese werden entweder in Form von Ampullen oder auch als sogenannte gebrauchsfertige Injektabila, z.B. als Fertigspritzen oder Einmalspritzen, daneben auch in Durchstechflaschen zur mehrmaligen Entnahme, hergerichtet. Die Verabreichung der Injektabila kann in Form der subkutanen (s.c.), intramuskulären (i.m.), intravenösen (i.v.) oder intrakutanen (i.c.) Applikation erfolgen. Die jeweils zweckmäßigen Injektionsformen können insbesondere als Lösungen, Kristallsuspensionen, nanopartikuläre oder kolloiddisperse Systeme, wie z.B. Hydrosole, hergestellt werden.

Die injizierbaren Zubereitungen können auch als Konzentrate hergestellt werden, welche mit wässrigen isotonischen Verdünnungsmitteln auf die gewünschte Wirkstoffdosierung eingestellt werden können. Weiterhin können sie auch als Pulver, wie z. B. Lyophylisate, hergestellt werden, die dann vorzugsweise unmittelbar vor der Applikation mit geeigneten Verdünnungsmitteln aufgelöst oder dispergiert werden.

Als Hilfs- und Trägerstoffe bei der Herstellung von injizierbaren Zubereitungen kommen *acqua sterilisata,* den pH-Wert beeinflussende Substanzen, wie z.B. organische und anorganische Säuren und Basen sowie deren Salze, Puffersubstanzen zur Einstellung des pH-Wertes, Isotonisierungsmittel, wie z.B. Natriumchlorid, Natriumhydrogencarbonat, Glucose und Fructose, Tenside bzw. oberflächenaktive Substanzen und Emulgatoren, wie z.B. Partialfettsäureester des Polyoxyethylensorbitans (Tween®), oder z.B. Fettsäureester des Polyoxyethylen (Cremophor®), fette Öle, wie z.B. Erdnussöl, Sojabohnenöl und Rizinussöl, synthetische Fettsäureester, wie z.B. Ethyloleat, Isopropylmyristat und Neutralöl (Miglycol®), sowie polymere Hilfsstoffe, wie z.B. Gelatine, Dextran, Polyvinylpyrrolidon, die Löslichkeit erhöhende Zusätze organischer Lösungsmittel, wie z.B. Propylenglycol, Ethanol, N,N-Dimethylacetamid, Propylenglycol oder komplexbildender Stoffe, wie z.B. Citraten und Harnstoff, Konservierungsmittel, wie z.B. Bonzolsäurehydroxypropyl- und methylester, Benzylalkohol, Antioxidantien, wie z.B. Natriumsulfit und Stabilisatoren, wie z.B. EDTA, in Betracht.

Die Verabreichung kann auch perlingual erfolgen.

Darreichungsformen zur verzögerten Freisetzung können ebenfalls verwendet werden.

Der Fachmann kann die jeweils geeigneten Arzneiformen in Einklang mit den Rezeptvorschriften und Verfahrensweisen auf Basis pharmazeutisch-physikalischer Grundlagen leicht erkennen und herstellen.

### Indikationsgebiete

Die erfindungsgemäße pharmazeutische Zusammensetzung mit einem Gehalt an Bulbophyllum kann bei allen Störungen des Herz-Kreislaufsystems, insbesondere bei Herzkrankheiten in der Familienanamnese, Organgefühl des Herzens, Beklemmung, Enge in der Brust, Schwere auf der Brust, Bradycardie, Tachycardie, Extrasystolie, Arrhythmie, Herzklopfen, Vorhofflimmern, Herzschmerzen, Herzstechen, Schmerzen am Sternum, Herzschmerzen mit Ausstrahlung in den linken Arm und Kribbeln im linken Arm, Angina pectoris, Stenose der Coronararterien, Herzinfarkt, Herzinsuffizienz mit Ödemen, Hypertrophie des Myocards bzw. des Septums, rechtsventrikulär vergrößertem Herzen, Ventrikel-Septum-Defekt, Vorhof-Septum-Defekt, primärer pulmonaler Hypertonie, Hypertonie, peripherer Zyanose der Akren, Lippenzyanose, Dyspnoe, Anstrengungsdyspnoe, Höhenunverträglichkeit, koronarbedingten Hitzewallungen, durch Herzinsuffizienz bedingten Durchblutungsstörungen der Extremitäten, Kälte der Extremitäten, durch Herzinsuffizienz bedingter körperlicher Schwäche, herabgesetzter Leistungsfähigkeit, Schwächezustand nach rheumatischem Fieber, Herzklappenstörungen, kardial bedingten Einschlafschwierigkeiten, Schlaflosigkeit, kardial bedingtem Husten, verabreicht werden.

### Pharmakologisch experimenteller Teil

Die erfindungsgemäße pharmazeutische Zusammensetzung wurde innerhalb von circa drei Jahren insgesamt 127 Patienten verabreicht. Von den Patienten waren 61 (49 %) weiblich. Von den 127 Patienten trat bei 82 Patienten (65 %) eine deutliche Besserung oder Heilung der Beschwerden auf. Unter diesen 82 trat bei 31 Patienten eine durchschlagende subjektive und objektive Besserung ihrer Beschwerden auf.

Bei allen 127 Patienten ist jeweils eine Einzelgabe angewendet worden. Im Zeitraum der Wirkung der Einzelgabe wurden keinerlei andere Medikamente verabreicht, auch keine Vitamine, Spurenelemente, Mineralien, Physiotherapeutika usw..

Bei den 82 Patienten, bei denen eine deutliche Besserung oder Heilung eintrat, war eine Wiederholung der Einzelgabe erst nach frühestens 7 Wochen notwendig. Die längste Wirkungsdauer lag bei 11 Monaten, das heißt, dass sich bei diesen Patienten erst nach 11 Monaten die Beschwerden wieder einstellten, so dass eine erneute Gabe erforderlich wurde. Die durchschnittliche Wirkdauer einer Einzelgabe liegt bei drei bis vier Monaten. Die Wirkungsdauer einer Einzelgabe ist abhängig vom Schweregrad der Erkrankung.

### Vergleich mit anderen Herztherapeutika in Bezug auf Wirkung, Nebenwirkung, Wirkungsdauer und Kosten

Verglichen mit der erfindungsgemäßen Bulbophyllum-haltigen pharmazeutischen Zusammensetzung hat kein anderes Herztherapeutikum eine solche Indikationsbreite und damit Wirkungsbreite.

Aufgrund der Tatsache, dass die Verabreichung in der Praxis erfolgt, und dann keine weitere Einnahme mehr nötig ist, bis die Beschwerden wieder auftreten, ist die Compliance bei keinem anderen Herztherapeutikum so hoch wie bei den erfindungsgemäßen Bulbophyllum-haltigen pharmazeutischen Zusammensetzungen.

Bei den meisten Patienten trat die Besserung ohne eine vorangegangene Verschlimmerung der Symptomatik auf. In circa 5 % der Fällen wurde eine für maximal drei bis fünf Tage anhaltende leichte Verschlimmerung der Symptomatik beobachtet. Bei etwa 15 % der Fälle trat in der dritten Woche nach der ersten Einnahme kurzfristig die Symptomatik auf, die aber nach einigen Stunden bzw. einigen Tagen ohne erneute Arzneigabe wieder abklang.

Bei keinem der untersuchten Patienten traten Nebenwirkungen auf.

Kein anderes Herztherapeutikum hat eine so lange Wirkungsdauer.

Außerdem sind bei dem erfindungsgemäßen Extrakt die Kosten für die Behandlung nur circa 1/6 der Kosten bei herkömmlichen Herztherapeutika, z. B. Niphedipin (Adalat).

Aufgrund der bei Herzkranken normalerweise üblichen Multimorbidität sind die Kosten für andere Beschwerden, die mit anderen Therapeutika therapiert werden, bei herkömmlichen Arzneimitteln noch hinzuzurechnen. Bei der Anwendung von Bulbophyllum war dieses Arzneimittel das einzige, das dem Patienten verabreicht wurde. Dadurch entfallen Kosten für andere Therapeutika, wodurch der Kostenvergleich noch erheblich stärker zugunsten von Bulbophyllum-haltigen pharmazeutischen Zusammensetzungen ausfällt.

## Patentansprüche

1. Verwendung von Bulbophyllum, gegebenenfalls in Verbindung mit geeigneten pharmazeutischen Hilfs- und Trägerstoffen zur Herstellung einer pharmazeutischen Zusammensetzung zur Behandlung von Störungen des Herz-Kreislaufsystems.

2. Verwendung gemäß Anspruch 1, wobei eine Bulbophyllum-Drogenzubereitung in Form eines alkoholischen Extrakts verwendet wird.

3. Verwendung gemäß Anspruch 2, wobei der Alkoholgehalt des alkoholischen Extrakts zwischen 80 und 100 Vol.-% beträgt.

4. Verwendung gemäß Anspruch 1, wobei eine Bulbophyllum-Drogenzubereitung in Form einer Verreibung mit Milchzucker verwendet wird.

5. Verwendung gemäß einem der vorhergehenden Ansprüche, wobei der Drogengehalt pro Einzelgabe im Bereich von 1 Nanogramm bis 1 Milligramm liegt.

6. Verwendung gemäß einem der vorhergehenden Ansprüche für die epidurale, perorale oder topische Verabreichung.

7. Verwendung gemäß einem der Ansprüche 1 bis 5 als Injektabila.

8. Verwendung gemäß Anspruch 7, wobei die Störung des Herz-Kreislaufsystems ausgewählt ist aus Herzkrankheiten in der Familienanamnese, Organgefühl des Herzens, Beklemmung, Enge in der Brust, Schwere auf der Brust, Bradycardie, Tachycardie, Extrasystolie, Arrhythmie, Herzklopfen, Vorhofflimmern, Herzschmerzen, Herzstechen, Schmerzen am Sternum, Herzschmerzen mit Ausstrahlung in den linken Arm und Kribbeln im linken Arm, Angina pectoris, Stenose der Coronararterien, Herzinfarkt, Herzinsuffizienz mit Ödemen, Hypertrophie des Myocards bzw. des Septums, rechtsventrikulär vergrößertem Herzen, Ventrikel-Septum-Defekt, Vorhof-Septum-Defekt, primärer pulmonaler Hypertonie, Hypertonie, peripherer Zyanose der Akren, Lippenzyanose, Dyspnoe, Anstrengungsdyspnoe, Höhenunverträglichkeit, koronarbedingten Hitzewallungen, durch Herzinsuffizienz bedingten Durchblutungsstörungen der Extremitäten, Kälte der Extremitäten, durch Herzinsuffizienz bedingter körperlicher Schwäche, herabgesetzter Leistungsfähigkeit, Schwächezustand nach rheumatischem Fieber, Herzklappenstörungen, kardial bedingten Einschlafschwierigkeiten, Schlaflosigkeit, kardial bedingtem Husten.

## Claims

1. Use of Bulbophyllum, optionally in combination with appropriate pharmaceutical excipients and carriers, for producing a pharmaceutical composition for the treatment of cardio-vascular disorders.

2. Use according to claim 1, wherein a drug preparation containing Bulbophyllum in the form of an alcoholic extract is used.

3. Use according to claim 2, wherein the alcohol content of the alcoholic extract ranges between 80 and 100 percent by volume.

4. Use according to claim 1, wherein a drug preparation containing Bulbophyllum is used in the form of a trituration with milk sugar.

5. Use according to one of the preceding claims, wherein the drug content per single dose ranges between 1 ng and 1 mg.

6. Use according to one of the preceding claims for epidural, peroral or topic application.

7. Use according to any one of claims 1 to 5 as injectibles.

8. Use according to claim 7, wherein the cardio-vascular disorder is selected from the group comprising cardiopathy in family history, unusual perception of the heart, cardiac condition related anxiety, chest tightness, chest pressure, bradycardia, tachycardia, extrasystoles, arrhythmia, palpitation, atrial fibrillation, cardialgia, stabbing heart pains, sternum pains, heart pains radiating into the left arm and tingling sensation in the left arm, angina pectoris, stenosis of the coronary arteries, myocardial infarct, cardiac insufficiency involving edemas, myocardial and septal hypertrophy, right ventricle cardiomegaly, ventricle septum defect, atrium septum defect, primary pulmonary hypertension, hypertonia, peripheral acral cyanosis, lip cyanosis, dyspnea, exertional dyspnea, altitude intolerance, coronaria related hot flushes, heart insufficiency related disturbance of blood flow in the limbs, cold limbs, physical weakness caused by heart insufficiency, reduced ability, weak condition following rheumatic fever, valvular disorders, cardiopathic difficulties falling asleep, insomnia, cardiopathic cough.

## Revendications

1. Utilisation de bulbophyllum, le cas échéant en liaison avec des excipients et des substances porteuses pharmaceutiques appropriés pour fabriquer une composition pharmaceutique destinée à traiter les troubles du système cardiovasculaire.

2. Utilisation selon la revendication 1, une préparation médicamenteuse de bulbophyllum étant utilisée sous la forme d'un extrait alcoolique.

3. Utilisation selon la revendication 2, la teneur en alcool de l'extrait alcoolique étant comprise entre 80 et 100 % en volume.

4. Utilisation selon la revendication 1, une préparation médicamenteuse de bulbophyllum étant utilisée sous la forme d'une trituration avec du lactose.

5. Utilisation selon l'une quelconque des revendications précédentes, la teneur en substance médicamenteuse par dose unique se situant dans une plage allant de 1 nanogramme à 1 milligramme.

6. Utilisation selon l'une quelconque des revendications précédentes pour l'administration épidurale, par voie buccale, ou topique.

7. Utilisation selon l'une quelconque des revendications 1 à 5 comme substance injectable.

8. Utilisation selon la revendication 7, les troubles du système cardiovasculaire étant choisis parmi les maladies cardiaques dans l'anamnèse familiale, sensation organique du coeur, oppression, poitrine serrée, lourdeur dans la poitrine, bradycardie, tachycardie, extrasystole, arythmie, palpitations, fibrillation atriale, douleurs cardiaques, picotements au coeur, douleurs au sternum, douleurs cardiaques avec irradiation dans le bras gauche et picotement dans le bras gauche, angine de poitrine, sténose des artères coronaires, infarctus du myocarde, insuffisance cardiaque avec oedèmes, hypertrophie du myocarde et/ou du septum, hypertrophie ventriculaire droite, communication interventriculaire, communication interauriculaire, hypertension pulmonaire primaire, hypertension, cyanose périphérique des extrémités, cyanose labiale, dyspnée, dyspnée à l'effort, mal d'altitude, bouffées de chaleur d'origine coronaire, troubles de la circulation sanguine au niveau des extrémités dus à l'insuffisance cardiaque, froid aux extrémités, faiblesse corporelle due à l'insuffisance cardiaque, forme physique diminuée, état de faiblesse suite à une fièvre rhumatismale, troubles des valvules du coeur, troubles de l'endormissement d'origine cardiaque, insomnie, toux d'origine cardiaque.
